(19) Europäisches Patentamt
European Patent Office
Office européen des brevets

(11) Veröffentlichungsnummer : **0 413 678 B1**

(12) # EUROPÄISCHE PATENTSCHRIFT

(45) Veröffentlichungstag der Patentschrift :
**02.11.94 Patentblatt 94/44**

(51) Int. Cl.$^5$ : **G01N 33/68,** G01N 33/52, G01N 31/22

(21) Anmeldenummer : **90890229.9**

(22) Anmeldetag : **26.07.90**

(54) **Verfahren zum Nachweis und zur Bestimmung von Humanserumalbumin in biologischen Flüssigkeiten mit Hilfe fluorogener Cyanin-Farbstoffe.**

(30) Priorität : **17.08.89 AT 1949/89**

(43) Veröffentlichungstag der Anmeldung :
**20.02.91 Patentblatt 91/08**

(45) Bekanntmachung des Hinweises auf die Patenterteilung :
**02.11.94 Patentblatt 94/44**

(84) Benannte Vertragsstaaten :
**DE FR GB**

(56) Entgegenhaltungen :
**EP-A- 0 141 298**
**EP-A- 0 287 745**
**ANALYTICAL CHEMISTRY, Band 58, Nr. 13, November 1986, Columbus, OH (US); K. SAUDA et al., Seiten 2649-2653**

(73) Patentinhaber : **Kessler, Manfred, Dr.**
**Lorenz-Vest-Weg 11**
**A-8041 Graz (AT)**
Patentinhaber : **Wolfbeis, Otto S., Dr.**
**Im Hoffeld 32**
**A-8046 Graz (AT)**

(72) Erfinder : **Kessler, Manfred, Dr.**
**Lorenz-Vest-Weg 11**
**A-8041 Graz (AT)**
Erfinder : **Wolfbeis, Otto S., Dr.**
**Im Hoffeld 32**
**A-8046 Graz (AT)**

(74) Vertreter : **Pinter, Rudolf, Dipl.-Ing.**
**Patentanwälte Klein & Pinter OEG**
**Fasangasse 49**
**A-1030 Wien (AT)**

**EP 0 413 678 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein neues fluorimetrisches Verfahren zur quantitativen Bestimmung von Humanserumalbumin in biologischen Flüssigkeiten. Die Methode basiert auf dem Befund, daß gewisse anionische Cyaninfarbstoffe in Anwesenheit von Humanserumalbumin eine starke Erhöhung der Fluoreszenzintensität erfahren und somit in einer verbesserten Bestimmungsmethode für Humanserumalbumin (im folgenden HSA genannt) verwendet werden können.

## Bestehende Verfahren

Verfahren des Standes der Technik zur spezifischen Bestimmung von Albumin in biologischen Flüssigkeiten sind in der Literatur beschrieben.

1) Beim sogenannten ELISA (enzyme-linked immunosorbent assay) handelt es sich um ein sehr sensitives und selektives immunologisch-enzymatisches Verfahren (Bergmeyer, Methods of Enzymatic Analysis, Third Edition (1986) vol. IX, p. 57).

2) Der RIA (radioimmuno-assay) ist ein sensitives und selektives radio-immunologisches Verfahren (J. Woo, M. Floyd, D. C. Cannon, B. Kahan, Radioimmunoassay for Urinary Albumin, Clin. Chem. 24, 1464-7 (1978)).

3) Die Immunoelektrophorese ist ein sensitives und selektives elektrophoretisch-immunologisches Verfahren (C. B. Laurell, Quantitative Estimation of Protein by Electrophoresis in Agarose Gel Containing Antibodies, Anal. Biochem. 15, 45-52 (1966)).

4) Weiters bekannt sind kinetische Bestimmungsverfahren auf Basis der intrinsischen Lipaseaktivität von HSA. Dabei wird die Geschwindigkeit der Spaltung eines synthetischen Fettsäureesters, welcher ein Enzymsubstrat darstellt, photometrisch oder fluorimetrisch verfolgt (A. R. Gürakar, O. S. Wolfbeis, A Sensitive Kinetic Assay of Serum Albumin Based on its Enzyme-Like hydrolytic Activity, Using a New chromogenic and Fluorogenic Substrate, Clin. Chim. Acta 172, 35-46 (1988).

5) Schließlich gibt es noch Verfahren, die auf der spektralen Veränderung von Farbstoffen beruhen (K. V. Waller, K. M. Ward, J. D. Mahan, D. K. Wismatt, Current Concepts in Proteinuria, Clin. Chem. 35 (5), 755-65 (1989)), voran das CBB-Verfahren: Der Farbstoff Coomassie Brilliant Blue bindet an HSA und schlägt dabei von Gelb nach Blau um Die Farbänderung beruht auf einer Änderung des $pK_a$-Wertes des Farbstoffes durch Bindung an HSA, wodurch der Farbstoff nach Bindung an HSA in die konjugierte Basenform übergeht. (M. M. Bradford, A Rapid and Sensitive Method for the Quantitation of Microgram Quantities of Protein Utilizing the Principle of Protein-Dye Binding, Anal. Biochem. 72, 248-54 (1976);

## Nachteile der bestehenden Verfahren

Diese bekannten Nachweisverfahren sind aus verschiedenen Gründen nicht voll befriedigend:

Verfahren des Standes der Technik, die auf einer spektralen Veränderung eines Farbstoffes bei Anwesenheit von HSA basieren, sind nicht sehr empfindlich (untere Nachweisgrenzen 10 - 100 mg/l) und außerdem unspezifisch, wodurch die Genauigkeit der Methoden vermindert wird.

Elektrophoretische Verfahren des Standes der Technik sind mit einem relativ hohen Zeit-, Arbeits- und Kostenaufwand verbunden, wenn geringe Mengen von HSA bestimmt werden sollen. Außerdem ist die Quantifizierung der Ergebnisse oft schwierig, da es in vielen Fällen zu keiner eindeutigen Trennung von HSA von Begleitproteinen kommt, was zu relativ großen Fehlern führen kann. Obwohl es einige Ansätze zur Verfeinerung der Auftrennungstechniken gibt (z. B. Isoelectric focusing (E. Meisinger, N. Gretz, M. Strauch: A New Possibility to Analyze Urinary Proteins: Isoelectrofocusing With Immobilized pH Gradients, Nephron 43 (1), 67-9 (1986)); Immunoelektrophorese (C. B. Laurell, Quantitative Estimation of Protein by Electrophoresis in Agarose Gel Containing Antibodies, Anal. Biochem. 15, 45-52 (1966))) bleiben diese aufwendigen Methoden auf Spezialanwendungen beschränkt.

Kinetische Verfahren des Standes der Technik, die auf der intrinsischen Lipaseaktivität von HSA beruhen, sind ebenfalls nicht sehr sensitiv und selektiv. Störend wirkt eine große Zahl von Begleitproteinen, besonders aber die Anwesenheit von Lipasen.

Immunologische Verfahren des Standes der Technik sind zwar sehr empfindlich und selektiv, aber arbeits-, kosten- und zeitaufwendig. Das zur Zeit modernste immunologische Verfahren des Standes der Technik, der enzyme-linked immunosorbent assay (ELISA) benötigt ca. 210 min. Inkubationszeit. Daher werden diese Verfahren nur für Spezialuntersuchungen eingesetzt.

Aufgabe der vorliegenden Erfindung ist es, ein neues Verfahren zur HSA-Bestimmung zur Verfügung zu stellen, das von den oben erwähnten Nachteilen des Standes der Technik frei ist. Diese Aufgabe wird mittels

der erfindungsgemäßen fluorimetrischen Bestimmung von HSA gelöst.

Die Erfindung beruht auf der Verwendung von anionischen Cyaninfarbstoffen der allgemeinen Formel (I)

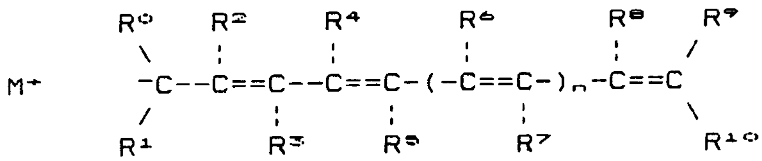

(I)

worin

| | |
|---|---|
| n | 0 oder 1 bedeutet, |
| $M^+$ | ein beliebiges einwertiges Kation sein kann, |
| $R^0$ | Cyano, Aryl oder COR (wobei R für Alkoxy, Alkyl oder Aryl steht) sein kann, und |
| $R^1$ | Cyano, Aryl oder COR (wobei R für Alkoxy, Alkyl oder Aryl steht) sein kann; worin weiters |
| $R^2$, $R^8$ | für Wasserstoff, Alkyl oder Cyano, |
| $R^3$, $R^5$, $R^6$, $R^7$ | für Wasserstoff oder Alkyl und |
| $R^4$ | für Wasserstoff, Halogen, Alkyl, Aryl, Aryloxy, Alkyloxy, oder $NR_2$ (wobei R Alkyl oder Aryl bedeutet) stehen, und weiters |
| $R^9$ | Cyano, Aryl, COR (wobei R für Alkoxy, Alkyl, oder Aryl steht), CO-NAB (wobei A für Alkyl, Aryl, oder Carbamoyl und B für Alkyl oder Aryl steht), Wasserstoff, Heterocyclyl, |
| $R^{10}$ | Cyano, Aryl, COR (wobei R für Alkoxy, Alkyl, oder Aryl steht), CO-NAB (wobei A für Alkyl, Aryl, oder Carbamoyl und B für Alkyl oder Aryl steht), Wasserstoff, oder Heterocyclyl bedeuten, und |

mindestens ein Rest von $R^0$, $R^1$, $R^9$, oder $R^{10}$ Cyano bedeutet.

$R^3$-$R^5$ kann auch für Glieder eines Ringes stehen, also z.B. für $-(CH_2)_2-$ oder $-(CH_2)_3-$.

Bevorzugt sind jene Verbindungen der allgemeinen Formel (I), in welcher

| | |
|---|---|
| n | 0 ist, |
| $R^2$ und $R^8$ | für Wasserstoff steht, |
| $R^3$-$R^5$ | eine Ethylen oder Propylengruppe und |
| $R^4$ | Chlor bedeutet. |

Besonders bevorzugt sind diejenigen Verbindungen der Formel (I) in der

| | |
|---|---|
| $R^0$, $R^1$ und $R^9$ | Cyano sind und |
| $R^{10}$ | für Cyano oder für einen gegebenenfalls durch Halogen substituierten Benzazol-2-yl-Rest steht. |

Die folgende Tab. 1 gibt eine Auswahl von anionischen Cyaninfarbstoffen, ohne damit aber die Zahl der zur Bestimmung geeigneten Fluoreszenzfarbstoffe einschränken zu wollen. Die entsprechenden Absorptionsmaxima sind in der letzten Spalte angeführt.

Tab.1. Substituentenmuster und Absorptionsmaxima (in nm) von typischen anionischen Cyaninfarbstoffen der allgemeinen Formel (I) für die fluorimetrische Bestimmung von HSA.

| Nr. | n | $R^0$ | $R^1$ | $R^2$ | $R^3$– $R^5$ | $R^4$ | $R^6$ | $R^7$ | $R^8$ | $R^9$ | $R^{10}$ | $\lambda_{max}$ [nm] |
|---|---|---|---|---|---|---|---|---|---|---|---|---|
| 1 | 0 | CN | CN | H | $-(CH_2)_2-$ | H | – | – | H | CN | CN | 584 |
| 2 | 0 | CN | CN | H | $-(CH_2)_2-$ | Ph | – | – | H | CN | CN | 594 |
| 3 | 0 | CN | CN | H | $-(CH_2)_2-$ | $OCH_3$ | – | – | H | CN | CN | 578 |
| 4 | 0 | CN | CN | H | $-(CH_2)_2-$ | $N(CH_3)_2$ | – | – | H | CN | CN | 540 |
| 5 | 0 | CN | CN | H | $-(CH_2)_2-$ | $CH_3$ | – | – | H | CN | CN | 595 |
| 6 | 0 | CN | CN | H | $-(CH_2)_2-$ | Cl | – | – | H | CN | CN | 599 |
| 7 | 0 | CN | COOEt | H | $-(CH_2)_2-$ | Cl | – | – | H | CN | COOH | 608 |
| 8 | 0 | CN | CN | H | $-(CH_2)_3-$ | $OCH_3$ | – | – | H | CN | CN | 564 |
| 9 | 0 | CN | CN | H | $-(CH_2)_3-$ | Cl | – | – | H | CN | CN | 580 |
| 10 | 0 | CN | COOEt | H | $-(CH_2)_3-$ | Cl | – | – | H | CN | COOEt | 588 |
| 11 | 0 | CN | CN | H | $-CH_2OCH_2-$ | H | – | – | H | CN | CN | 547 |
| 12 | 0 | CN | CN | H | H, H | H | – | – | H | CN | CN | 538 |
| 13 | 0 | CN | CN | H | H, H | Cl | – | – | H | CN | CN | 564 |
| 14 | 0 | CN | CN | CN | H, H | H | – | – | CN | CN | CN | 632 |
| 15 | 0 | CN | CN | CN | –CH==CH– | H | – | – | CN | CN | CN | 574 |
| 16 | 0 | CN | CN | H | H, H | H | – | – | H | CN | COOEt | 542 |
| 17 | 0 | CN | CN | $CH_3$ | H, H | H | – | – | $CH_3$ | CN | CN | 572 |
| 18 | 1 | CN | CN | H | H, H | H | H | H | H | CN | CN | 632 |
| 19 | 0 | CN | CN | H | $-(CH_2)_2-$ | Cl | – | – | H | CN | 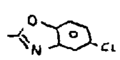 | 648 |
| 20 | 0 | CN | CN | H | $-(CH_2)_2-$ | Cl | – | – | H | CN | 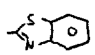 | 658 |
| 21 | 0 | CN | CN | H | $-(CH_2)_2-$ | Cl | – | – | H | CN | 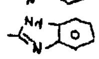 | 627 |
| 22 | 0 | CN | CN | H | $-(CH_2)_2-$ | Cl | – | – | H | CN | CO–Ph | 597 |
| 23 | 0 | CN | CN | H | $-(CH_2)_2-$ | Cl | – | – | H | CN | $CONHCONH_2$ | 600 |

Die erfindungsgemäßen Cyaninfarbstoffe sind wasserlösliche, violett bis grün gefärbte Verbindungen, die im orangen bis roten Spektralbereich fluoreszieren. Die Fluoreszenz in rein wässriger Lösung ist relativ schwach. Wird zu dieser Lösung jedoch HSA zugegeben, verschiebt sich das Fluoreszenzmaximum zu größeren Wellenlängen und die Fluoreszenzintensität wird drastisch erhöht. Dieser Effekt ist so stark, daß schon Spuren von HSA eine beträchtliche Signaländerung bewirken. Überraschenderweise bewirken Begleitproteine von HSA in biologischen Flüssigkeiten keinen solchen Effekt. So verursachen z. B. γ-Globuline, die bei den meisten Nierenerkrankungen im Harn mengenmäßig nach HSA an zweiter Stelle stehen,weniger als 0.1 % der

Signaländerung einer gleichen Menge HSA. Deshalb kann die Methode zur spezifischen HSA-Bestimmung ohne vorheriger Abtrennung der Begleitproteine angewendet werden.

Bedingt durch die über weite Bereiche geltende lineare Beziehung zwischen Anregungsenergie und Fluoreszenzintensität kann das Signal und somit die Empfindlichkeit der Methode durch Laseranregung beträchtlich erhöht werden. Ein besonders günstiges Signal/Rausch Verhältnis wird erreicht, indem man die Rotverschiebung des Excitationsmaximums des Fluoreszenzfarbstoffes bei Bindung an HSA zusätzlich ausnützt.

---> Abb. 1

Abbildung 1. zeigt die Anregungsspektren (gemessen bei einer Emissionswellenlänge von 640 nm) des Fluoreszenzfarbstoffes Nr. 6 aus Tab. 1. Bei Abwesenheit von HSA (Kurve 1) ist die Fluoreszenzintensität sehr klein. Wird jedoch HSA zugegeben (Kurve 2), tritt sowohl eine drastische Erhöhung der Fluoreszenzintensität als auch eine Rotverschiebung des Excitationsmaximums auf. Aus den Spektren in Abbildung 1 geht hervor, daß bei Wahl eines Helium-Neon-Lasers als Anregungslichtquelle die nicht an HSA gebundenen Farbstoffmoleküle bei 633 nm nur wenig effizient angeregt werden. Dadurch wird die Untergrundfluoreszenz weitgehend reduziert. Durch Bindung an das HSA verschiebt sich das Anregungsmaximum langwellig, sodaß der Farbstoff nun durch die Laserlinie weit effizienter angeregt wird. Dadurch kommt es zu einer weitgehend selektiven Anregung nur der an HSA gebundenen Form, und die Untergrundfluoreszenz der ohnehin nur schwach fluoreszierenden freien Form des Fluoreszenzfarbstoffes wird dadurch praktisch vernachlässigbar gering.

In analoger Weise können auch andere Fluoreszenzfarbstoffe der allgemeinen Formel (I) eingesetzt und durch entsprechende spektral vorteihafte Anregungslichtquellen wie z. B. Diodenlaser oder Leuchtdioden angeregt werden.

Durch diese Technik wird die untere Nachweisgrenze für HSA im wesentlichen nur durch die geringe Haltbarkeit und die schwierige Handhabung sehr verdünnter HSA-Eichlösungen wegen der Adsorptionsprozesse an Gefäßwänden limitiert und ist somit jener der empfindlichsten Verfahren des Standes der Technik vergleichbar.

In einer weiteren Ausgestaltung der Erfindung wird nicht die Änderung der Fluoreszenzintensität als Folge der Bindung des Fluoreszenzfarbstoffes an HSA als analytischer Parameter herangezogen, sondern die Änderung der Fluoreszenzdepolarisation bzw. der Fluoreszenzabklingzeit. Es wird gefunden, daß die Fluoreszenz der erfindungsgemäßen Fluoreszenzfarbstoffe in wässriger Lösung von pH = 7.40 vollkommen depolarisiert vorliegt, aber durch Bindung an HSA polarisiert wird. Durch Messung der auftretenden polarisierten Fluoreszenz hat man somit eine weitere Möglichkeit zum qualitativen und quantitativen Nachweis von HSA. Da sich weiters auch die Fluoreszenzabklingzeit des Fluoreszenzfarbstoffes durch Bindung an HSA verändert, hat man schließlich auch in der zeitaufgelösten Fluorimetrie eine Methode zum qualitativen und quantitativen Nachweis von HSA in der Hand.

Gegenüber Bestimmungsverfahren für HSA des Standes der Technik weist das neue Verfahren mit Hilfe der erfindungsgemäßen Cyaninfarbstoffe der allgemeinen Formel (I) wesentliche Vorteile auf:

1. Die Methode weist einen sehr geringen Kosten- und Zeitaufwand auf, da die Bestimmung auf der Zugabe der Reagenslösung zu einer Probelösung und anschließender Messung der Fluoreszenzintensität beruht.
2. Die Empfindlichkeit der Methode kann durch Variation der Farbstoffkonzentration und der apparativen Parameter auf jede aktuelle Probe abgestimmt werden und ist zumindest ebenso gut wie die der empfindlichsten Verfahren des Standes der Technik.
3. Die Methode ist sehr selektiv, sodaß auch ein großer überschuß an Fremdproteinen, wie sie etwa in biologischen Flüssigkeiten vorkommen, nicht stört. Daher entfällt ein vorgeschalteter Trennschritt.
4. Durch die Kompatibilität der erfindungsgemäßen Fluoreszenzfarbstoffe mit optischen Komponenten aus der Halbleitertechnik (Leuchtdioden oder Diodenlaser als Lichtquellen bzw. Photodioden als Detektoren) werden alle Vorteile der Halbleitertechnik, wie niedrige Spannungsversorgung, kompakte Bauweise, geringer Stromverbrauch, hohe Lebensdauer und Betriebssicherheit, voll übernommen.
5. Durch die langwellige Fluoreszenzanregung bzw. - emission wird die störende kurzwellige Untergrundfluoreszenz von biologischem Material nicht miterfaßt, sodaß die hohe Empfindlichkeit der Fluorimetrie voll ausgenützt wird.

Die erfindungsgemäßen Fluoreszenzfarbstoffe können nach den im folgenden beschriebenen Verfahren oder nach J. L. Slominskii, S. V. Popov, A. Ilchenko, A. I. Tolmachev, Zh. Organ. Khimii, 21, 1294 (1985) bzw. M. Strell, W. B. Braunbruck, W. F. Fühler, O. Hüber, Liebigs Ann. Chem. 587, 177 (1954) hergestellt werden.

Zur Herstellung der neuen erfindungsgemäßen anionischen Cyaninfarbstoffe der Formel (I) werden zunächst Derivate von Dialdehyden der Formel (II)

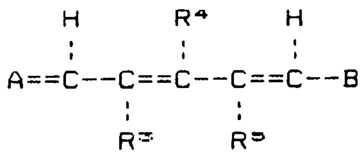 (II)

worin

A: ($X^-$ $^+N(CH_3)_2$) (worin $X^-$ für einen Säurerest (bevorzugt Perchlorat) steht), oder Sauerstoff und

B: $-N(CH_3)_2$, $-OCH_3$ oder -OH bedeuten und weiters

$R^3$, $R^4$ und $R^5$ die obengenannte Bedeutung besitzen, mit aktiven Methylenverbindungen der Formel (III),

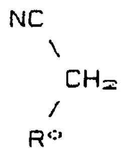 (III)

worin

$R^0$ die obengenannte Bedeutung hat, in Gegenwart eines basischen Kondensationsmittels umgesetzt und das erhaltene Zwischenprodukt der Formel (IV) isoliert.

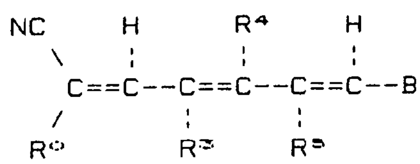 (IV)

Darin steht

B für $-N(CH_3)_2$, $OCH_3$ oder -OH und

$R^0$, $R^3$, $R^4$ und $R^5$ besitzen die obengenannte Bedeutung.

Im einem zweiten Schritt kann das Zwischenprodukt der Formel (IV) mit einer weiteren aktiven Methylenkomponente der Formel (V),

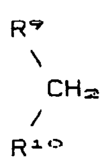 (V)

worin $R^9$ und $R^{10}$ die obengenannte Bedeutung haben, zum anionischen Cyaninfarbstoff der Formel (I) umgesetzt werden. Diese Reaktion erfolgt wieder in Gegenwart von Basen.

Sind die beiden Methylenkomponenten (III) und (V) identisch, kann auf die Isolierung des Zwischenproduktes der Formel (IV) verzichtet werden und die Reaktion als Eintopfsynthese ausgeführt werden.

Als Basen eignen sich Pyridin, tertiäre aliphatische Amine, quartäre Ammonium, Alkali- und Erdalkalihydroxide. Im Fall besonders aktiver Methylenkomponenten (z.B. Malonodinitril) wird die Reaktion bevorzugt in Pyridin durchgeführt. Weniger aktive Methylenkomponenten werden bevorzugt in Pyridin/Triethylamin-Mischungen oder mit Hilfe von Tetramethylammoniumhydroxid umgesetzt.

Beide Reaktionsschritte werden vorzugsweise in Gegenwart eines Lösungs- oder Verdünnungsmittels im Temperaturbereich 20 bis 100 °C durchgeführt. Als solche eignen sich die bereits oben angeführten flüssigen organischen Basen, vorzugsweise Pyridin. Bei Verwendung von Hydroxiden als Basenkomponenten werden vorzugsweise absolutes Ethanol oder Methanol verwendet.

Um die Reaktion des Dialdehydderivates (II) im ersten Schritt mit zwei Molekülen (III) zu vermeiden, legt man zweckmäßig einen überschuß von (II) vor und gibt die Komponente (III) nach und nach zu, gegebenenfalls

vorgelöst in einem der oben angeführten Lösungsmittel.

Die Ausgangsverbindungen der Formel (II) sind bekannt. (L. Berlin und O. Riester in: Houben-Weyl, Methoden der Organischen Chemie, 4.Auflage, Band 5/1d, Georg Thieme Verlag Stuttgart 1972; G. A. Reynolds und K. H. Drexhage, J. Org. Chem. 42, 885 (1977)).

In den folgenden Beispielen wird das Verfahren an Hand der Synthese zweier Fluoreszenzfarbstoffe aus Tabelle 1 und der Bestimmung von HSA in Harnen mit Hilfe eines dieser Farbstoffe erläutert, ohne aber damit die Anwendungsbreite des Verfahrens eingrenzen zu wollen.

Beispiel 1. Synthese eines HSA-sensitiven, mit einem Helium-Neon-Laser anregbaren Fluoreszenzfarbstoffes (Nr. 6 aus Tabelle 1). Ausführung der Synthese als Eintopfsynthese:

Zu einer Mischung von 6.3 g (20 mmol) N-((2-Chlor-3-(dimethylamino)-methylen)-1-cyclopenten-1-yl)-methylen)-N,N-dimethylammonium perchlorat (II) (A: $ClO_4^-$ $^+(CH_3)_2N$- ; B: $-N(CH_3)_2$ ; $R^4$: Cl ; $R^3$-$R^5$: $-(CH_2)_2-$ ) und 3.3 g (50 mmol) Malonodinitril werden 5 ml Pyridin zugesetzt. Die Mischung wird sofort dunkelblau und erstarrt bald zu einem Kristallbrei. Die Suspension wird noch 1 h auf 70-80 °C erwärmt und über Nacht in den Kühlschrank gestellt. Danach werden 20 ml Diethylether zugegeben und filtriert. Der Niederschlag wird mit Diethylether gewaschen und im Exsikkator über $P_4O_{10}$ getrocknet, um Pyridinreste zu entfernen. Ausbeute: 6.7 g (68% d. Th.). Der Farbstoff kann ohne weitere Reinigung im erfindungsgemäßen Bestimmungsverfahren verwendet werden.

Beispiel 2. Synthese eines HSA-sensitiven Fluoreszenzfarbstoffes (Nr. 19 aus Tabelle 1), der mit einem 670-680 nm Diodenlaser angeregt werden kann.

Zu einer Lösung von 7.0 g (22.3 mmol) N-((2-Chlor-3-(dimethylamino)-methylen)-1-cyclopenten-1-yl)-methylen)-N,N-dimethylammonium perchlorat (II) (A: $ClO_4^-$ $^+(CH_3)_2N$- ; B: $-N(CH_3)_2$ ; $R^4$: Cl ; $R^3$-$R^5$: $-(CH_2)_2-$ ) in ca. 30 ml Pyridin wird eine vorher bereitete Lösung von 1.2 g (18 mmol) Malonodinitril in 5 ml Pyridin unter gutem Rühren im Laufe von 10 min. zugetropft. Dann wird noch 15 min auf 70 °C erwärmt und die abgekühlte Lösung mit Petrolether versetzt. Der Niederschlag wird abgesaugt, mit insgesamt 500 ml Methanol/Wasser 1:1 (v/v) und mit Diethylether gewaschen. Ausbeute: 3.6 g violette Nadeln. Zur Reinigung wird aus Toluol umkristallisiert. Fp: 212 °C.
Im zweiten Schritt werden 0.16 g (0.69 mmol) dieses Produktes und 0.14 g (0.73 mmol) 5-Chlor-2-cyanomethylenbenzoxazol in ca. 15 ml Methanol unter gelindem Erwärmen gelöst. Dann werden 0.13 g (0.7 mmol) Tetramethylammonium hydroxid (x 5 $H_2O$) zugegeben und noch 2 h bei Raumtemperatur gerührt. Die erhaltene Suspension wird mit 50 ml Diethylether versetzt und abgesaugt. Das Produkt wird zwei mal mit Diethylether gewaschen. Ausbeute: 0.15 g (49% d. Th.). Fp: 221 °C. Der Farbstoff kann ohne weitere Reinigung zur erfindungsgemäßen Anwendung verwendet werden.

Beispiel 3. Bestimmung von HSA in pathologischen Harnen neben verschiedenen Begleitproteinen. Verwendung des Farbstoffes von Beispiel 1 und eines 2 mW Helium-Neon-Lasers als Anregungslichtquelle. Meßbereich: 2-4000 mg HSA/l

1) Stammlösung des Fluoreszenzfarbstoffes:

Der Fluoreszenzfarbstoff wird in wasserfreiem Isopropanol gelöst. Damit diese Stammlösung haltbar ist, wird die Farbstoffkonzentration so eingestellt, daß die Extinktion bei 599 nm ca. 1.5 beträgt. Die Lösung ist im Dunkeln aufzubewahren.

2) Puffer

Phosphatpuffer von pH = 7.40, mit NaCl auf physiologische Ionenstärke von I = 160 mmolar eingestellt.

3) HSA-Eichlösung

10 mg lyophilisiertes HSA werden in 100 ml Puffer gelöst.

4) Erstellung der Eichkurve

Je 0.0, 0.5, 1, 2, 3 und 4 ml der HSA-Eichlösung werden mit der Pufferlösung auf 9.5 ml aufgefüllt. Diese

Lösungen werden mit 0.5 ml der Farbstoffstammlösung versetzt, kurz umgeschwenkt und sogleich im Fluorimeter unter Helium-Neon-Laseranregung vermessen. Die analytische Größe ist die Fluoreszenzintensität bei 630 nm. Die so erhaltene Eichkurve ist in Abb. 2 dargestellt.

---> Abb. 2

5) Bestimmung von HSA im Harn

Die Bestimmung von HSA im Harn erfolgt analog zur Erstellung der Eichgerade. Dabei ist zu beachten, daß sedimentreiche bzw. trübe Harne zweckmäßig vorher filtriert oder zentrifugiert werden. Die zur Bestimmung eingesetzte Harnmenge richtet sich im wesentlichen nach dem Gehalt an HSA. Es ist zweckmäßig so zu verdünnen, daß der Meßpunkt auf der Eichgerade liegt.

6) Ergebnisse der HSA-Bestimmung einiger ausgewählter Harne und Vergleich mit immunologisch/nephelometrisch erhaltenen Daten:

Harn 1: sehr trüber, hochpathologischer Harn

Begleitprotein: Bence-Jones 3200 mg/l

HSA-Gehalt: nach dem erfindungsgemäßen Verfahren: 60 mg/l

Immunologisch/Nephelometrisch: 65 mg/l

Harn 2: klarer Harn, leichte Mikroalbuminurie feststellbar

HSA-Gehalt: nach dem erfindungsgemäßen Verfahren: 30 mg/l

Immunologisch/Nephelometrisch: 32.5 mg/l

Harn 3: sedimentreicher Harn, keine pathologische Albuminurie feststellbar.

HSA-Gehalt: nach dem erfindungsgemäßen Verfahren: 18 mg/l

Immunologisch/Nephelometrisch: 15.5 mg/l

Harn 4: klarer Harn, keine pathologische Albuminurie feststellbar.

HSA-Gehalt: nach dem erfindungsgemäßen Verfahren: 10 mg/l

Turbidimetrisch: unter der Nachweisgrenze

Harn 5: klarer Harn, hochpathologisch (Albuminurie)

Begleitprotein: Globuline

HSA-Gehalt: nach dem erfindungsgemäßen Verfahren: 3100 mg/l

Immunologisch/Nephelometrisch : 3025 mg/l

Harn 6: synthetischer Kontrollharn

bestehend aus: γ-Globulin: 59 mg/l

HSA-Gehalt: nach dem erfindungsgemäßen Verfahren: 0 mg/l

**Patentansprüche**

1. Verfahren zum fluoreszenz-optischen Nachweis und zur Bestimmung von Humanserumalbumin in biologischen Flüssigkeiten, dadurch gekennzeichnet, daß man
   a) eine gegebenenfalls verdünnte biologische Probe mit einem anionischen Cyaninfarbstoff der allgemeinen Struktur (I) versetzt

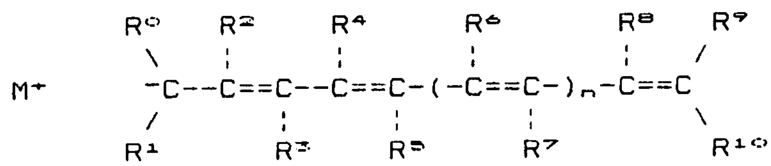

(I)

worin

| | |
|---|---|
| n | 0 oder 1 bedeutet, |
| $M^+$ | ein beliebiges einwertiges Kation sein kann, |
| $R^0$ | Cyano, Aryl oder COR (wobei R für Alkoxy, Alkyl oder Aryl steht) sein kann, und |
| $R^1$ | Cyano, Aryl oder COR (wobei R für Alkoxy, Alkyl oder Aryl steht) sein kann; worin weiters |
| $R^2$, $R^8$ | für Wasserstoff, Alkyl oder Cyano, |
| $R^3$, $R^5$, $R^6$, $R^7$ | für Wasserstoff oder Alkyl und |
| $R^4$ | für Wasserstoff, Halogen, Alkyl, Aryl, Aryloxy, Alkyloxy, oder $NR_2$ (wobei R Alkyl oder Aryl bedeutet) stehen, und weiters |
| $R^9$ | Cyano, Aryl, COR (wobei R für Alkoxy, Alkyl, oder Aryl steht), CO-NAB (wobei A für Alkyl, Aryl, oder Carbamoyl und B für Alkyl oder Aryl steht), Wasserstoff, Heterocyclyl, |
| $R^{10}$ | Cyano, Aryl, COR (wobei R für Alkoxy, Alkyl, oder Aryl steht), CO-NAB (wobei A für Alkyl, Aryl, oder Carbamoyl und B für Alkyl oder Aryl steht), Wasserstoff, oder Heterocyclyl bedeuten, |

   und mindestens ein Rest von $R^0$, $R^1$, $R^9$, oder $R^{10}$ Cyano bedeutet.

   $R^3$-$R^5$ können auch für Glieder eines Ringes stehen, also z.B. für $-(CH_2)_2-$ oder $-(CH_2)_3-$.

   b) daß man die Intensität der Fluoreszenz, oder ihre Depolarisation oder Abklingzeit bestimmt, und

   c) die durch die Bindung an Humanserumalbumin hervorgerufene Änderung der Fluoreszenzeigenschaften zur quantitativen Bestimmung des Humanserumalbumins mit Hilfe bekannter oder vorab ermittelter Eichkurven heranzieht.

2. Verfahren nach Anspruch 1), dadurch gekennzeichnet, daß ein anionischer Fluoreszenzfarbstoff der allgemeinen Formel I verwendet wird, in welchem

| | |
|---|---|
| n | = 0, |
| $R^2$ und $R^8$ | für Wasserstoff stehen, |
| $R^3$ und $R^5$ | durch eine Ethylen -oder Propylenbrücke verknüpft sind, und |
| $R^4$ | für Chlor steht, |
| $R^0$, $R^1$, und $R^9$ | Cyano, und |
| $R^{10}$ | Cyano oder einen 2-Benzazolylrest bedeuten. |

3. Verfahren nach Ansprüchen 1)-2), dadurch gekennzeichnet, daß die Fluoreszenzanregung mit einem Helium-Neon-Laser oder einem Diodenlaser erfolgt.

4. Verfahren nach Ansprüchen 1)-3), dadurch gekennzeichnet, daß die Fluoreszenzanregung bei jener Wellenlänge erfolgt, bei der das Verhältnis der Extinktionen des an Humanserumalbumin gebundenen zu der des ungebundenen Fluoreszenzfarbstoffes ein Maximum ist.

## Claims

1. Method of fluorescence-optical indication and determination of human serum albumin in biological fluids, characterised in that one

   a) reacts a biological sample, possibly diluted, with an anionic cyanine dye of the general structure (I)

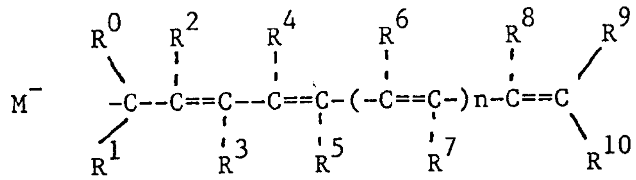

(I)

in which

| | |
|---|---|
| n | signifies 0 or 1 |
| $M^-$ | can be any desired monovalent cation, |
| $R^0$ | can be cyano, aryl or COR (where R stands for alkoxy, alkyl or aryl), and |
| $R^1$ | can be cyano, aryl or COR (where R stands for alkoxy, alkyl or aryl); and wherein furthermore |
| $R^2$, $R^3$ | stand for hydrogen, alkyl or cyano, |
| $R^3$, $R^5$, $R^6$, $R^7$ | stand for hydrogen or alkyl and |
| $R^4$ | stands for hydrogen, halogen, alkyl, aryl, aryloxy, alkyloxy or $NR_2$ (where R signifies alkyl or aryl) and further |
| $R^9$ | signifies cyano, aryl, COR (where R stands for alkoxy, alkyl or aryl), CO-NAB (where A stands for alkyl, aryl or carbamoyl and B for alkyl or aryl), hydrogen, heterocyclyl, |
| $R^{10}$ | signifies cyano, aryl, COR (where R stands for alkoxy, alkyl or aryl), CO-NAB (where A stands for alkyl, aryl or carbamoyl and B for alkyl or aryl), hydrogen or heterocyclyl, |

and at least one residue of $R^0$, $R^1$, $R^9$ or $R^{10}$ signifies cyano.

$R^3$ -$R^5$ could also stand for members of a ring, thus for example for $-(CH_2)_2-$ or $-(CH_2)_3-$.

b) that the intensity of the fluorescence, or its depolarisation, or decay period is determined, and

c) the alteration of the fluorescence characteristics resulting from the binding to human serum albumin is obtained for quantitatively determining the human serum albumin with the aid or known or previously obtained standard curves.

2. Method according to Claim 1), characterised in that an anionic fluorescence dye of the general formula (I) is employed, in which

| | |
|---|---|
| n | = 0, |
| $R^2$ and $R^8$ | stand for hydrogen, |
| $R^3$ and $R^5$ | are linked by an ethylene or propylene bridge, and |
| $R^4$ | stands for chlorine, |
| $R^0$, $R^1$ and $R^7$ | signifies cyano, and |
| $R^{10}$ | signifies cyano or a 2-benzazolyl residue. |

3. Method according to Claims 1)-2), characterised in that the fluorescence excitation is obtained using a helium-neon laser or a diode laser.

4. Method according to Claims 1)-3), characterised in that the fluorescence excitation takes place at that

wavelength at which the ratio of the extinctions of the fluorescence dye bound to the human serum albumin to that of the unbound fluorescence dye is a maximum.

## Revendications

1. Procédé d'obtention d'une fluorescence optique et de détermination d'albumine de sérum humain dans des fluides biologiques, caractérisé en ce que:

   a) on traite un échantillon biologique, le cas échéant dilué avec un colorant cyanine anionique présentant la formule générale (I):

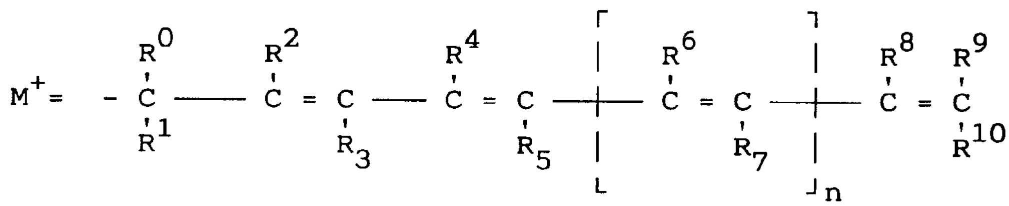

dans laquelle:

| | |
|---|---|
| n | est égal à 0 ou 1, |
| $M^-$ | est un cation monovalent quelconque, |
| $R^0$ | est un groupe cyano, aryle ou COR (R étant un radical alcoxy, alkyle ou aryle), |
| $R^1$ | est un groupe cyano, aryle ou COR (R étant un radical alcoxy, alkyle ou aryle), |
| $R^2$, $R^8$ | sont des atomes d'hydrogène ou des groupes alkyles ou cyano, |
| $R^3$, $R^5$, $R^6$, $R^7$ | sont des atomes d'hydrogène ou des groupes alkyles, |
| $R^4$ | est un atome d'hydrogène ou d'halogène ou un radical alkyle, aryle, aryloxy, alkyloxy ou $NR_2$ (R étant un radical alkyle ou aryle), |
| $R^9$ | est un groupe cyano, aryle ou COR (R étant un radical alcoxy, alkyle ou aryle), CO-NAB (A étant un radical alkyle, aryle ou carbamoyle et B étant un radical alkyle ou aryle), un atome d'hydrogène ou un groupe hétérocyclique, |
| $R^{10}$ | est un groupe cyano, aryle ou COR (R étant un radical alcoxy, alkyle ou aryle), CO-NAB (A étant un radical alkyle, aryle ou carbamoyle et B étant un radical alkyle ou aryle), un atome d'hydrogène ou un groupe hétérocyclique, |

et au moins un des groupes $R^0$, $R^1$, $R^9$ ou $R^{10}$ est un groupe cyano,

$R^3$ - $R^5$ pouvant également former un cycle, tel par exemple $-(CH_2)_2$ ou $-(CH_2)_3$

b) on détermine l'intensité de la fluorescence ou sa dépolarisation ou sa durée de désactivation, et

c) la modification des propriétés de fluorescence provoquée par la liaison avec l'albumine de sérum humain et nécessaire pour la détermination quantitative de l'albumine de sérum humain est déterminée à l'aide des courbes d'étalonnage connues ou déterminées auparavant.

2. Procédé selon la revendication 1, caractérisé en ce que le colorant fluorescent anionique présente la formule générale (I) dans laquelle:

| | |
|---|---|
| n | est égal à 0, |
| $R^2$, $R^8$ | sont des atomes d'hydrogène, |
| $R^3$ et $R^5$ | sont liés par des ponts éthylène ou propylène |
| $R^4$ | est du chlore, |
| $R^0$, $R^1$ et $R^9$ | sont des groupes cyano |
| $R^{10}$ | est un groupe cyano ou un résidu 2-benzazolyle. |

3. Procédé selon la revendication 1 ou 2, caractérisé en ce que la stimulation de la fluorescence est produite par un laser hélium-néon ou un laser à diode.

4. Procédé selon l'une quelconque des revendications 1 à 3, caractérisé en ce que la stimulation de la fluorescence est produite par toute longueur d'onde dont le rapport de l'extinction liée à l'albumine de sérum humain au colorant fluorescent non lié est un maximum.

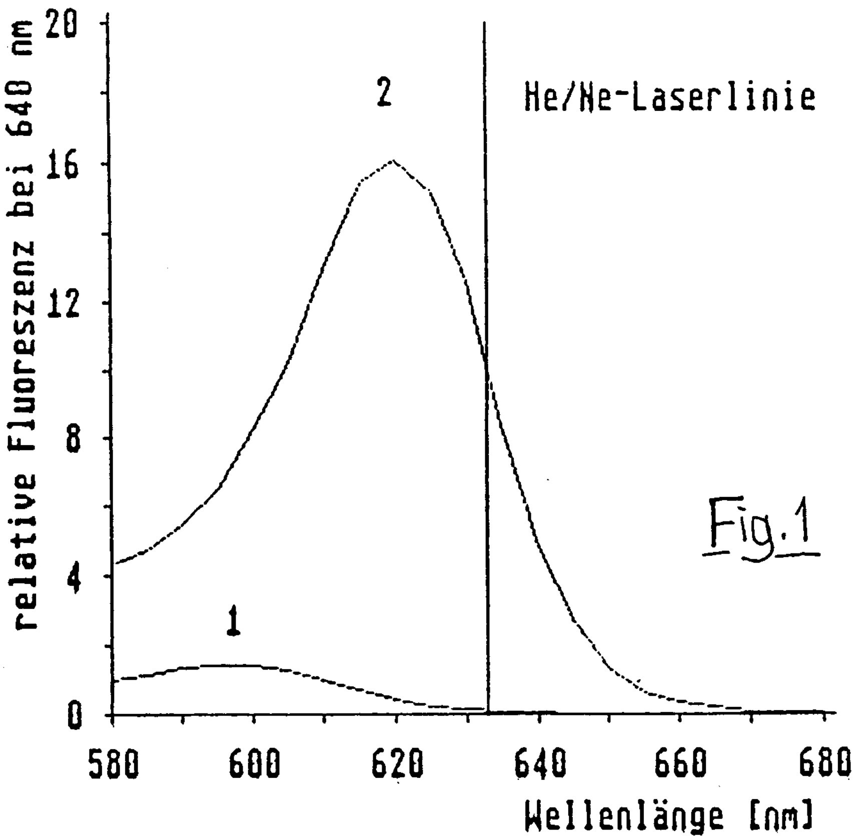
relative Fluoreszenz bei 640 nm
20
16
12
8
4
0
2
1
He/Ne-Laserlinie
580
600
620
640
660
680
Wellenlänge [nm]

Fig.1

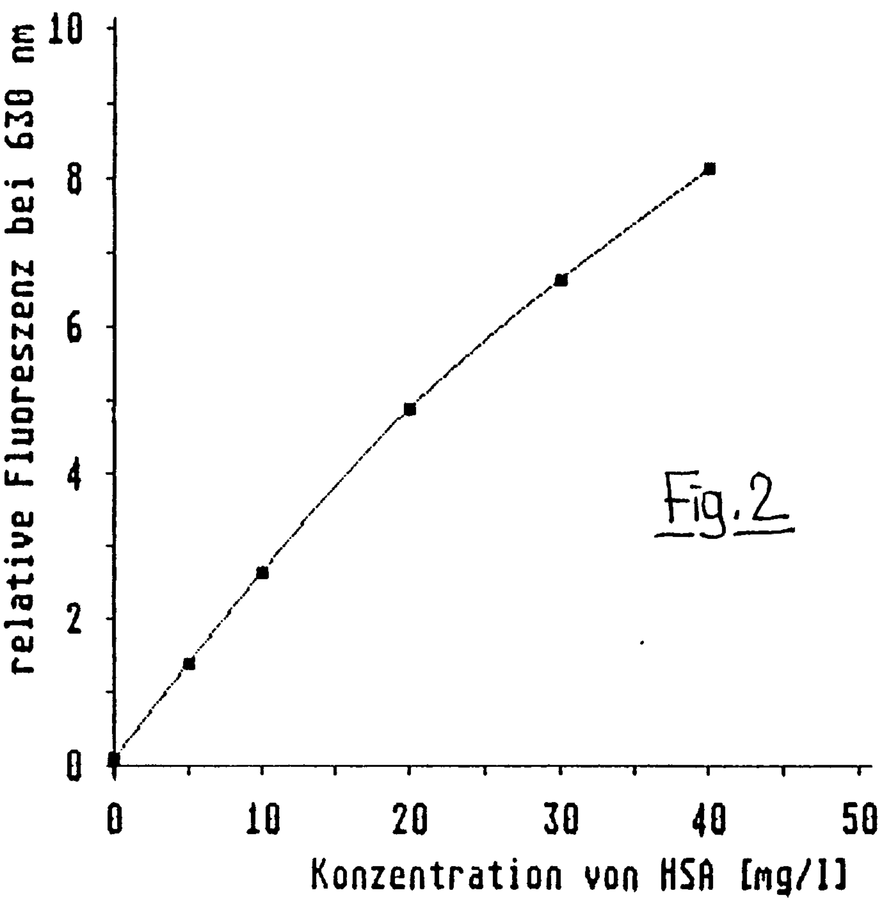
relative Fluoreszenz bei 630 nm
10
8
6
4
2
0
0
10
20
30
40
50
Konzentration von HSA [mg/l]

Fig. 2